(19) Europäisches Patentamt
European Patent Office
Office européen des brevets

(11) Publication number: 0 035 899
B1

# (12) EUROPEAN PATENT SPECIFICATION

(45) Date of publication of patent specification: 30.05.84

(51) Int. Cl.$^3$: A 61 K 7/06

(21) Application number: 81300968.5

(22) Date of filing: 09.03.81

## (54) Hair conditioning composition and method of conditioning hair therewith.

(30) Priority: 10.03.80 US 128436
22.12.80 US 218376
17.02.81 US 234297

(43) Date of publication of application:
16.09.81 Bulletin 81/37

(45) Publication of the grant of the patent:
30.05.84 Bulletin 84/22

(84) Designated Contracting States:
AT BE CH DE FR GB IT LI LU NL SE

(56) References cited:
FR-A-1 563 677
GB-A-1 152 972
GB-A-2 025 228
US-A-3 964 500

CHEMICAL ABSTRACTS, vol. 80, no. 8, 25th February 1974, page 261, column 2, no. 40939w Columbus, Ohio, U.S.A.

(73) Proprietor: THE PROCTER & GAMBLE COMPANY
301 East Sixth Street
Cincinnati Ohio 45202 (US)

(72) Inventor: Bolich, Raymond Edward, Jr.
7201 Striker Road
Maineville Ohio 45039 (US)
Inventor: Hartsough, Lloyd Bruch
3826 Lincoln Road
Cincinnati Ohio 45239 (US)
Inventor: Cothran, Philip Earl
6941 RobVern Drive
Cincinnati Ohio 45239 (US)

(74) Representative: Brooks, Maxim Courtney et al
Procter & Gamble (NTC) Limited Whitley Road
Longbenton
Newcastle-upon-Tyne NE12 9TS (GB)

EP 0 035 899 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention is related to hair conditioning compositions which can condition hair while not leaving an undesirable residue.

Some hair is naturally difficult to manage and to comb in the wet state, while other types of hair have become so as a result of the various treatments to which the hair has been subjected, such treatments including permanent waving, dyeing, tinting, frequent teasing, exposure to various atmospheric conditions, etc. Hair that has been subjected to an oxidizing condition, e.g. treatments with hydrogen peroxide or atmospheric oxygen photocatalyzed by sunlight, is especially difficult to comb in the wet state.

Many products are marketed for use at home and in the beauty shops to obviate and overcome the difficulty encountered in wet-combing. However, these products usually affect the hair's ability to maintain a set and stay clean.

Some of the cream rinse products which provide good wet-combing of the hair contain quaternary ammonium compounds. Their use produces a dilemma, their effectiveness as a wet-combing aid is proportional to the amount of cream rinse retained on the hair but the rate at which the hair appears and feels dirty is also proportional to that amount.

The use of volatile actives in hair care products is known. US—A—3,577,528 (equivalent to GB—A—1,152,972) discloses two phase hair conditioners comprising an aqueous phase which contains a quaternary compound and a hydrocarbon or fluorinated hydrocarbon water immiscible phase. The compositions may also contain a polyvinylpyrrolidone-type polymer. Chemical Abstracts, Vol 80, Abstract No. 40939w, 1974 discloses a three phase hair conditioner comprising a $C_{10}$—$C_{20}$ isoparaffin oil phase, a polyalkyleneglycol phase and an aqueous phase. US—A—3,932,610 discloses a shampoo composition which may contain a volatile hydrocarbon solvent. US—A—3,818,105, discloses hair conditioners containing a $C_{12}$ to $C_{14}$ isoparaffinic hydrocarbon fraction. South African Patent Application 666421 discloses hair conditioners containing volatile silicones.

While these references disclose compositions which contain components of the type present in the compositions of the present invention they are not entirely satisfactory. Thus compositions in immiscible multiphase form tend to be less acceptable with consumers than compositions in the form of stable emulsions. Also polymers such as the polyvinylpyrrolidones can have a tendency to deposit on the hair. The present invention therefore provides hair conditioner compositions in emulsion form which overcome problems associated with prior compositions.

According to the invention, there is provided a hair conditioning composition comprising from 1% to 13% by weight of a liquid hair conditioning agent selected from $C_{10}$—$C_{16}$ straight or branched chain hydrocarbons, cyclic polydimethylsiloxanes having from 3 to 7 silicon atoms and linear polydimethylsiloxanes having from 3 to 9 silicon atoms, from 0.05% to 4% by weight of a cationic hair conditioning agent and water, characterized in that the composition is in the form of an emulsion and additionally comprises from 0.1% to 8% by weight of a substantially nonionic water-soluble polymer having a viscosity in excess of 20 Pa.s (200 poises) at a shear rate of $10^{-2}$ $sec^{-1}$ while having a viscosity of less than 0.9 Pa.s (9 poises) at a shear rate of 500 $sec^{-1}$, the water-soluble polymer being selected from polyoxyethylene, guar gum, methyl cellulose, methyl hydroxypropyl cellulose, locust bean gum, hydroxyethyl cellulose, hydroxypropyl guar gum, hydroxypropyl cellulose, starch, hydroxyethyl amylose, starch amylose and mixtures thereof.

The compositions of the present invention comprise the above described essential components and may additionally contain several optional components. Each of the components is discussed in detail below.

*Liquid Hair Conditioning Agent*

The hydrocarbon and polydimethylsiloxane agents useful in the present compositions have a boiling point in the range of 99°C to 260°C and have a solubility in water of less than 0.1%. The hydrocarbons are either straight or branched chain and contain from 10 to 16, preferably from 12 to 16 carbon atoms. Examples of suitable hydrocarbons are decane, dodecane, tetradecane, tridecane and mixtures thereof.

The polydimethylsiloxanes useful in the compositions of the present invention are either a cyclic or a linear polydimethylsiloxane. The number of silicon atoms in the cyclic silicones is from 3 to 7, preferably 4 or 5.

The general formula for such silicones is

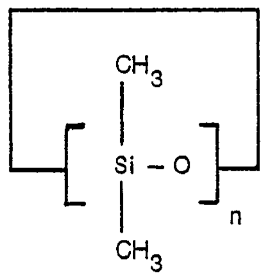

wherein n = 3—7

The linear polydimethylsiloxanes have from 3 to 9 silicon atoms and have the general formula

$$(CH_3)_3Si—O—[Si(CH_3)_2—O]_n—Si(CH_3)_3 \quad n = 1—7$$

Silicones of the above type, both cyclic and linear, are offered by Dow Corning Corporation, Dow Corning 344, 345 and 200 fluids, Union Carbide, Silicone 7202 and Silicone 7158, and Stauffer Chemical, SWS—03314.

The linear polydimethylsiloxanes generally have viscosities of less than 5 centistokes at 25°C. while the cyclic materials have viscosities less than 10 centistokes. A description of volatile silicones is found in Todd and Byers, "Volatile Silicone Fluids for Cosmetics", *Cosmetics and Toiletries,* Vol. 91, January, 1976, pp. 27—32.

The liquid hair conditioning agent is present in the compositions of this invention at a level of from 1% to 13% by weight, preferably from 2% to 10% by weight. The polydimethylsiloxanes are the preferred agents.

*Nonionic Thickening Agent (Nonionic Water-soluble Polymer)*

The water soluble thickening agent useful in the present compositions is a substantially nonionic water soluble polymer. The polymers are those which exhibit viscosities exceeding 20 Pa.s (200 poises) at $10^{-2}sec^{-1}$, when their viscosities at 500 $sec^{-1}$ are less than 0.9 Pa.s (9 poises). Preferred are those polymers which show about a 100 fold increase in viscosity over the shear rate range of 500 to $10^{-2}sec^{-1}$. The polymers are selected from polyoxyethylene, guar gum, methylcellulose, methyl hydroxypropyl cellulose, hydroxyethyl cellulose, locust bean gum, hydroxypropyl guar gum, hydroxypropyl cellulose, starch, hydroxyethyl amylose and starch amylose and mixtures thereof. Preferred polymers are guar gum and hydroxypropyl guar gum.

The water soluble thickening agent is present in the present compositions at a level of from 0.1% to 8% by weight, preferably from 0.5% to 6.0% by weight.

*Cationic Hair Conditioning Agent*

The cationic hair conditioning agent of the present compositions may be either a quaternary ammonium salt or the salt of a fatty amine.

Quaternary ammonium salts have the formula:

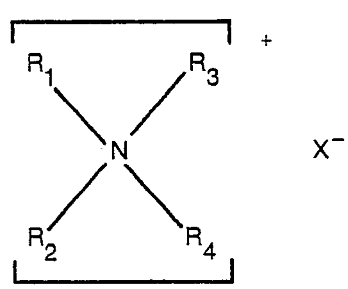

Wherein $R_1$ is hydrogen, an aliphatic group of from 1 to 22 carbon atoms, or aromatic, aryl or alkaryl groups having from 12 to 22 carbon atoms; $R_2$ is an aliphatic group having 1—22 carbon atoms; $R_3$ and $R_4$ are each alkyl groups of from 1 to 3 carbon atoms, and X is an anion selected from halogen, acetate, phosphate, nitrate and methyl sulfate radicals. The aliphatic groups may contain, in addition to carbon and hydrogen atoms, ether linkages as well as amido groups among other groups.

Preferred quaternary ammonium salts are the dialkyl dimethyl ammonium chlorides, wherein the alkyl groups have from 12 to 22 carbon atoms and are derived from long-chain fatty acids, such as hydrogenated tallow. The term "tallow" refers to fatty alkyl groups derived from tallow fatty acids. Such fatty acids give rise to quaternary compounds wherein $R_1$ and $R_2$ have predominately from 16 to 18 carbon atoms. The term "coconut" refers to fatty acid groups from coconut oil fatty acids. The coconut-alkyl $R_1$ and $R_2$ groups have from 8 to 18 carbon atoms and predominate in $C_{12}$ to $C_{14}$ alkyl groups.

Representative examples of quaternary ammonium salts of the invention include ditallow dimethyl ammonium chloride; ditallow dimethyl ammonium methyl sulfate; dihexadecyl dimethyl ammonium chloride; di(hydrogenated tallow) dimethyl ammonium chloride; dioctadecyl dimethyl ammonium chloride; dieicosyl dimethyl ammonium chloride; didocosyl dimethyl ammonium chloride; di(hydrogenated tallow) dimethyl ammonium acetate; dihexadecyl diethyl ammonium chloride; dihexadecyl dimethyl ammonium acetate; ditallow dipropyl ammonium phosphate; ditallow dimethyl ammonium nitrate; di(coconutalkyl)dimethyl ammonium chloride; and stearyl dimethyl benzyl ammonium chloride.

Other quaternary ammonium salts useful herein are the compounds of the formula

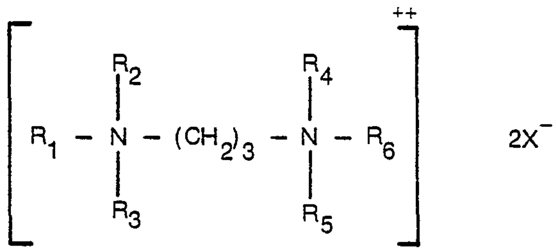

wherein $R_1$ is an aliphatic group having 16 to 22 carbon atoms, $R_2$, $R_3$, $R_4$, $R_5$ and $R_6$ are the same or different and are selected from H and alkyls having 1—4 carbon atoms and X is an anion as above defined. Tallow propanediammonium dichloride is an example of this quaternary ammonium salt.

Quaternary imidazolinium salts have the formula

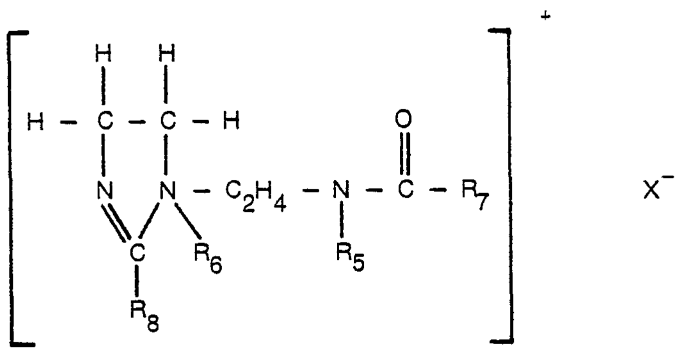

wherein $R_6$ is an alkyl group containing from 1 to 4, preferably from 1 to 2 carbon atoms, $R_5$ is an alkyl group containing from 1 to 4 carbon atoms or a hydrogen atom, $R_8$ is an alkyl group containing from 1 to 22, preferably at least 15 carbon atoms or a hydrogen atom, $R_7$ is an alkyl group containing from 8 to 22, preferably at least 15 carbon atoms, and X is an anion, preferably chloride.

Other suitable anions include those disclosed with reference to the quaternary ammonium salts described hereinbefore.

Particularly preferred are those imidazolinium salts in which both $R_7$ and $R_8$ are alkyls of from 12 to 22 carbon atoms, e.g., 1-methyl-1-[(stearylamido)ethyl)]-2-heptadecyl-4, 5-dihydroimidazolinium chloride; 1-methyl-1-[(palmitylamido)ethyl]-2-octadecyl-4,5-dihydroimidazolinium chloride and 1-methyl-1-[(tallowamido)-ethyl]-2-tallow-imidazolinium methyl sulfate.

Included as a suitable cationic hair conditioner herein are salts of fatty amines. As used herein the amines may be primary, secondary or tertiary but the alkyl, substituted and unsubstituted groups preferably have from 12—22 carbon atoms. Preferred are the primary and secondary amines with the primary being the most preferred. Diamines having a long chain alkyl group may also be used. Examples of amines suitable for use include dimethyl stearamine, dimethyl soyamine, stearylamine, soyamine, myristylamine, tridecylamine, ethyl stearylamine, N-tallow propanediamine, ethoxylated (5 moles E.O.) stearylamine dihydroxyethyl stearylamine and arachidylbehenylamine. The anions of the salts include those mentioned previously for the quaternary ammonium salts. Specific amine salts include stearylamine hydrochloride, soyamine chloride, stearylamine formate and N-tallow propanediamine dichloride.

The cationic hair conditioning agent is present at a level of from 0.05% to 4% by weight, preferably from 0.1% to 2% by weight, in the compositions of the present invention.

*Water*

Water, preferably, distilled water is the final essential component of the present compositions. Water makes up the remainder of the compositions and is generally present at a level of up to 95% by weight, preferably from 75% to 90% by weight.

*Optional Components*

In addition to the above described essential components the present compositions may contain a wide variety of optional components. The optional components can be materials which are soluble in the liquid hair conditioning agent phase, in the aqueous phase or not soluble in either phase.

Included among materials soluble in the liquid hair conditioning agent phase are hydrophobic polymers such as polyvinyl isobutyl ether, waxes such as cetyl alcohol and paraffin, and oils such as mineral oil, isopropyl myristate and non-volatile silicones. Agents which are soluble in the aqueous phase include acrylamide and polyoxyethylene resins.

Among other optional components are dyes, perfumes, opacifiers, pearlescent aids, buffers, preservatives, antioxidants, and antidandruff aids such as zinc pyrithione and sulfur.

There are many approaches for making the hair conditioning compositions of the present invention. The approach selected should provide particles of the liquid hair conditioning agent in the range of from 1 to 10 $\mu$m (microns). This can be accomplished by adding the liquid hair agent phase to the aqueous phase or vice versa. Suitable processes are shown in the Examples.

The hair conditioning compositions of the present invention are preferably used as a rinse on freshly shampooed hair. The compositions are used in an amount of from 1g. to 60g., preferably from 5g. to 30g. and is then rinsed from the hair.

The following examples further described and demonstrate embodiments within the scope of the present invention.

Unless otherwise indicated, all percentages herein are by weight.

Example I

The following composition was prepared:

| | |
|---|---|
| Dow Corning Fluid 344[1] (cyclic polydimethylsiloxane having 4 dimethyl siloxane groups) | 6.00% |
| Jaguar HP—60[2] (hydroxypropyl guar gum) | 1.00 |
| Adogen 432[3] (dicetyl dimethyl ammonium chloride) | 0.40 |
| Formaldehyde | 0.02 |
| Distilled Water | q.s. 100.00% |

[1] Supplied by Dow Corning Corporation.
[2] Supplied by Stein-Hall & Co., Inc.
[3] Supplied by Sherex Chemical Company

The above composition was prepared by dispersing 10 grams of Jaguar HP—60 in 926 grams of distilled water using a Lightnin®Mixer. The mixture was heated to about 65°C. to fully dissolve and hydrate the Jaguar gum. Four grams of Adogen 432 were then added and dispersed. The polydimethylsiloxane fluid was added next and dispersed. A high shear mixer, an Ultra Turrax® Model 45S4 dispersator supplied by Tekmer Company, was finally used to further reduce the polydimethylsiloxane particle size to 1 to 10 microns. The formaldehyde was added as the batch cooled to room temperature.

Example II

The following composition of the present invention is prepared in a manner similar to that described in Example I.

| | |
|---|---|
| Union Carbide 7158 Silicone Fluid[1] (Cyclic polydimethylsiloxane fluid having 5 dimethyl siloxane groups) | 8.00% |
| Supercol U[2] (guar gum) | 0.90 |
| Ditallow dimethyl ammonium chloride[3] | 1.00 |
| Perfume | 0.50 |
| Ethanol | 8.00 |
| Distilled water | q.s. 100.00% |

[1] Supplied by Union Carbide Corporation.
[2] Supplied by Henkel, Inc.
[3] Supplied by Sherex Chemical Company.

Example III

The following composition of the present invention is prepared in a manner similar to that described in Example I.

| | |
|---|---|
| Dodecane[1] | 10.00% |
| Jaguar HP—60 | 1.75 |
| Ditallow methyl amine hydrochloride[2] | 0.25 |
| Polyox WSR—205[3] | 0.10 |
| Methyl paraben[4] | 0.20 |
| Propyl paraben[4] | 0.10 |
| Perfume | 0.80 |
| Distilled Water | q.s. 100.00% |

[1] Supplied by Humphrey Chemical Company.
[2] Supplied by Sherex Chemical Company.
[3] Supplied by Union Carbide Corporation.
[4] Supplied by Inolex Corporation.

Example IV

The following composition is prepared in a manner similar to that described in Example I except that the polydimethylsiloxane and the Lutonal are preblended.

| | |
|---|---|
| Dow Corning Fluid 345[1] (cyclic polydimethylsiloxane having 5 dimethyl siloxane groups) | 7.00% |
| Lutonal 1C 115[2] (polyvinyl isobutyl ether) | 0.02 |
| Corn starch powder[3] | 3.50 |
| Stearyl benzyl dimethyl ammonium chloride[4] | 0.30 |
| Ethanol | 7.00 |
| Distilled water | q.s. 100.00% |

[1] Supplied by Dow Corning Corporation
[2] Supplied by BASF
[3] Supplied by CPC International, Inc.
[4] Supplied by Hexcel-Fine Organics.

Example V

The following composition was prepared:

| | |
|---|---|
| Union Carbide 7158 Silicone Fluid | 8.000% |
| Lutonal 1C 115 | 0.008 |
| Jaguar HP—60 | 1.100 |
| Ethanol | 8.000 |
| Adogen 442[1] (90% active) | 0.240 |
| Perfume | 0.500 |
| Distilled water | q.s. 100.000% |

[1] Ditallow dimethyl ammonium chloride supplied by Sherex Chemical Company.

The above composition was prepared by placing 76.08 grams of the polydimethylsiloxane, 400 grams of distilled water and 4 grams of a stock solution consisting of 2% Lutonal 1C 115 in polydimethylsiloxane into a mix tank. The stock solution had been prepared earlier by dissolving 2 grams of the ether in 98 grams of the polydimethylsiloxane and mixing with a magnetic stirrer for 24 hours. A pre-mix was prepared by dispersing 2.4 grams of the quaternary and 11 grams of Jaguar HP—60 in 80 grams of ethanol. This was mixed with a magnetic stirrer for 10 minutes at ambient temperature. This pre-mix was then added to the main mix tank and sheared with an Ultra Turrax® Mode 45S4 for five minutes. To the batch were then added 421 grams of distilled water and 5 grams of perfume. A Lightnin® mixer was used to complete mixing the batch for 15 minutes.

## Claims

1. A hair conditioning composition comprising from 1% to 13% by weight of a liquid hair conditioning agent selected from $C_{10}$—$C_{16}$ straight or branched chain hydrocarbons, cyclic polydimethylsiloxanes having from 3 to 7 silicon atoms and linear polydimethylsiloxanes having from 3 to 9 silicon atoms, from 0.05% to 4% by weight of a cationic hair conditioning agent and water, characterized in that the composition is in the form of an emulsion and additionally comprises from 0.1% to 8% by weight of a substantially nonionic water-soluble polymer having a viscosity in excess of 20 Pa.s (200 poises) at a shear rate of $10^{-2}$ $sec^{-1}$ while having a viscosity of less than 0.9 Pa.s (9 poises) at a shear rate of 500 $sec^{-1}$, the water-soluble polymer being selected from polyoxyethylene, guar gum, methyl cellulose, methyl hydroxypropyl cellulose, locust bean gum, hydroxyethyl cellulose, hydroxypropyl guar gum, hydroxypropyl cellulose, starch, hydroxyethyl amylose, starch amylose and mixtures thereof.

2. A hair conditioning composition according to Claim 1 characterized in that the liquid hair conditioning agent is a hydrocarbon selected from decane, dodecane, tridecane, tetradecane and mixtures thereof.

3. A hair conditioning composition according to Claim 1 characterized in that the liquid hair conditioning agent is a cyclic polydimethylsiloxane having either 4 or 5 dimethylsiloxane groups.

4. A hair conditioning composition according to any of Claims 1 to 3 characterized in that the amount of liquid hair conditioning agent is from 2% to 10% by weight, the amount of nonionic, water soluble polymer is from 0.05% to 6.0% by weight and the amount of cationic hair conditioning agent is from 0.1% to 2% by weight.

5. A hair conditioning composition according to any of Claims 1 to 4 characterized in that the substantially nonionic, water soluble polymer is selected from guar gum, hydroxypropyl guar gum and, mixtures thereof.

6. A hair conditioning composition according to any of Claims 1 to 5 characterized in that the cationic hair conditioning agent is a quaternary ammonium compound.

7. A hair conditioning composition according to any of Claims 1 to 6 characterized in that the nonionic, water soluble, polymer is hydroxypropyl guar gum, the cationic hair conditioning agent is ditallow dimethyl ammonium chloride, and the liquid hair conditioning agent is a cyclic polydimethylsiloxane having 5 dimethylsiloxane groups.

8. A method of conditioning hair characterized by

I. applying from 1 g to 60 g of a composition according to any of Claims 1 to 7 to freshly shampooed hair; and

II. rinsing the composition from the hair.

## Patentansprüche

1. Eine Zusammensetzung zum Konditionieren der Haare, enthaltend 1 Gew.-% bis 13 Gew.-% eines flüssigen Haarkonditioniermittels, ausgewählt aus gerad- oder verzweigtkettigen $C_{10}$—$C_{16}$-Kohlenwasserstoffen, cyclischen Polydimethylsiloxanen mit 3 bis 7 Siliciumatomen and linearen Polydimethylsiloxanen mit 3 bis 9 Siliciumatomen, 0,05 Gew.-% bis 4 Gew.-% eines kationischen Haarkonditioniermittels und Wasser, dadurch gekennzeichnet, daß die Zusammensetzung in der Form einer Emulsion vorliegt und zusätzlich 0,1 Gew.-% bis 8 Gew.-% eines im wesentlichen nichtionischen, wasserlöslichen Polymers enthält, das eine Viskosität von mehr als 20 Pa.s (200 Poise) bei einer Schergeschwindigkeit von $10^{-2}$ $s^{-1}$ hat, während es eine Viskosität von weniger als 0,9 Pa.s (9 Poise) bei einer Schergeschwindigkeit von 500 $s^{-1}$ aufweist, und das wasserlösliche Polymer aus Polyoxyethylen, Guargummi, Methylcellulose, Methylhydroxypropylcellulose, Johannisbrotgummi, Hydroxyethylcellulose, Hydroxypropyl-Guargummi, Hydroxypropylcellulose, Stärke Hydroxyethylamylose, Stärkeamylose und Mischungen davon ausgewählt ist.

2. Eine Zusammensetzung zum Konditionieren der Haare nach Anspruch 1, dadurch gekennzeichnet, daß das flüssige Haarkonditioniermittel ein cyclisches Polydimethylsiloxan ist, das entcan, Tridecan, Tetradecan und Mischungen davon ausgewählt ist.

3. Eine Zusammensetzung zum Konditionieren der Haare nach Anspruch 1, dadurch gekennzeichnet, daß das flüssige Haarkonditioniermittel ein cyclisches Polydimethylsiloxan ist, das entweder 4 oder 5 Dimethylsiloxangruppen aufweist.

4. Eine Zusammensetzung zum Konditionieren der Haare nach irgendeinem der Ansprüche 1 bis 3, dadurch gekennzeichnet, daß die Menge an flüssigem Haarkonditioniermittel 2 Gew.-% bis 10 Gew.-% beträgt, die Menge an nichtionischem, wasserlöslichem Polymer 0,05 Gew.-% bis 6,0 Gew.-% ausmacht und die Menge an kationischem Haarkonditioniermittel 0,1 Gew.-% bis 2 Gew.-% beträgt.

5. Eine Zusammensetzung zum Konditionieren der Haare nach irgendeinem der Ansprüche 1 bis 4, dadurch gekennzeichnet, daß das im wesentlichen nichtionische, wasserlösliche Polymer aus Guargummi, Hydroxypropyl-Guargummi und Mischungen davon ausgewählt ist.

6. Eine Zusammensetzung zum Konditionieren der Haare nach irgendeinem der Ansprüche 1 bis 5, dadurch gekennzeichnet, daß das kationische Haarkonditioniermittel eine quaternäre Ammoniumverbindung ist.

7. Eine Zusammensetzung zum Konditionieren der Haare nach irgendeinem der Ansprüche 1 bis 6, dadurch gekennzeichnet, daß das nichtionische, wasserlösliche Polymer Hydroxypropyl-Guargummi ist, das kationische Haarkonditioniermittel Ditalgalkyldimethylammoniumchlorid ist und das flüssige Haarkonditioniermittel ein cyclisches Polydimethylsiloxan, das 5 Dimethylsiloxangruppen aufweist, ist.

8. Ein Verfahren zum Konditionieren der Haare, gekennzeichnet durch:

I. das Aufbringen von 1 g bis 60 g einer Zusammensetzung nach irgendeinem der Ansprüche 1 bis 7 auf frisch shampoonierte Haare und

II. das Abspülen der Zusammensetzung aus den Haaren.

**Revendications**

1. Composition de conditionnement des cheveux, comprenant de 1% à 13% en poids d'un agent liquide de conditionnement des cheveux choisi parmi les hydrocarbures en $C_{10}$ à $C_{16}$ à chaîne droite ou ramifiée, les polydiméthylsiloxanes cycliques ayant de 3 à 7 atomes de silicium et les polydiméthylsiloxanes linéaires ayant de 3 à 9 atomes de silicium de 0,05% à 4% en poids d'un agent cationique de conditionnement des cheveux et de l'eau, caractérisée en ce que la composition se présente sous la forme d'une émulsion et comprend en outre de 0,1% à 8% en poids d'un polymère soluble dans l'eau, pratiquement non-ionique, ayant une viscosité supérieure à 20 Pa.s (200 poises) pour un taux de cisaillement de $10^{-2}$ $s^{-1}$, tout en ayant une viscosité inférieure à 0,9 Pa.s (9 poises) pour un taux de cisaillement de 500 $s^{-1}$, le polymère soluble dans l'eau étant choisi parmi le polyoxyéthylène, la gomme de guar, la méthylcellulose, la méthylhydroxypropylcellulose, la gomme de caroubes, l'hydroxyéthylcellulose, la gomme d'hydroxypropyl-guar, l'hydroxypropylcellulose, l'amidon, l'hydroxyéthylamylose, l'amylose d'amidon et leurs mélanges.

2. Composition de conditionnement des cheveux selon la revendication 1, caractérisé en ce que l'agent liquide de conditionnement des cheveux est un hydrocarbure choisi parmi le décane, le docécane, le tridécane, le tétradécane et leurs mélanges.

3. Composition de conditionnement des cheveux selon la revendication 1, caractérisée en ce que l'agent liquide de conditionnement des cheveux est un polydiméthylsiloxane cyclique ayant 4 ou 5 groupes diméthylsiloxane.

4. Composition de conditionnement des cheveux selon l'une quelconque des revendications 1 à 3, caractérisée en ce que la proportion de l'agent liquide de conditionnement des cheveux est de 2% à 10% en poids, la proportion du polymère non ionique soluble dans l'eau est de 0,05% à 6,0% en poids et la quantité de l'agent cationique de conditionnement des cheveux est de 0,1% à 2% en poids.

5. Composition de conditionnement des cheveux selon l'une quelconque des revendications 1 à 4, caractérisée en ce que le polymère soluble dans l'eau pratiquement non ionique est choisi parmi la gomme de guar, la gomme d'hydroxypropyl-guar et leurs mélanges.

6. Composition de conditionnement des cheveux selon l'une quelconque des revendications 1 à 5, caractérisée en ce que l'agent cationique de conditionnement des cheveux est un ammonium quaternaire.

7. Composition de conditionnement des cheveux selon l'une quelconque des revendications 1 à 6, caractérisée en ce que le polymère soluble dans l'eau, non ionique est la gomme d'hydroxypropyl-guar, l'agent cationique de conditionnement des cheveux est le chlorure de di(suif)-diméthylammonium, et l'agent liquide de conditionnement des cheveux est un polydiméthylsiloxane cyclique ayant 5 groupes diméthylsiloxane.

8. Procédé de conditionnement des cheveux, caractérisé en ce qu'il consiste:

I. à appliquer de 1 g à 60 g d'une composition selon l'une quelconque des revendications 1 à 7 sur des cheveux fraîchement laves; et

II. à éliminer par rinçage la composition des cheveux.